# DEMANDE DE BREVET EUROPEEN

(11) **EP 3 023 955 A1**
(43) Date de publication de la demande: **25.05.2016**
(21) Numéro de dépôt: 15195109.2
(22) Date de dépôt: 18.11.2015
(51) Int. Cl.: G08B 21/04, A61N 1/39, G06F 19/00, H04L 29/08, H04W 4/02, A61B 5/00, A61N 1/372, G08B 27/00, A61B 5/11

(54) **PROCEDE DE GESTION D'UN PARC DE DISPOSITIFS DE SECOURS, SERVEUR DE GESTION D'UN PARC DE DISPOSITIFS DE SECOURS ET DISPOSITIF DE SECOURS ASSOCIES**

(30) Priorité: 20.11.2014 FR 1461277
(71) Demandeur: BULL SAS, 78340 Les Clayes sous Bois (FR)
(72) Inventeur: PELLETIER, Benoît, 38960 Saint Etienne de Crossey (FR); ZENG EYINDANGA, Landry Stéphane, 38100 Grenoble (FR); BOURGEOIS, Christian, 38960 Saint Etienne de Crossey (FR)
(74) Mandataire: Cabinet Plasseraud

(57) **Abrégé**

L'invention concerne un procédé de gestion d'un parc de dispositifs de secours (3), comprenant : une étape de localisation (11) d'un appareil de surveillance (2) porté par une victime (1) d'un problème de santé, une étape de localisation (12), dans le parc de dispositifs de secours (3), d'au moins un dispositif de secours (3) proche de l'appareil de surveillance (2) localisé, une étape d'alarme (15) signalant au moins la position du dispositif de secours (3) localisé, une étape de guidage (18) indiquant, au niveau du dispositif de secours (3) localisé, au moins la position de l'appareil de surveillance (2) localisé.

## Description

### DOMAINE DE L'INVENTION

L'invention concerne un procédé de gestion d'un parc de dispositifs de secours, un serveur de gestion d'un parc de dispositifs de secours, et un dispositif de secours. Le dispositif de secours est avantageusement un défibrillateur cardiaque.

### CONTEXTE DE L'INVENTION

Selon un art antérieur, dans lequel les dispositifs de secours sont des défibrillateurs cardiaques, il est connu un système dans lequel des villes et/ou des organismes publics et/ou des sociétés déploient dans leurs quartiers, établissements et locaux respectifs, des défibrillateurs cardiaques en libre-service.

Lorsqu'une personne est alors victime d'un accident cardiaque, les témoins doivent identifier un secouriste parmi eux ou en rechercher un aux alentours au plus vite. Une fois qu'il a été identifié, ce secouriste commence à rechercher un défibrillateur à proximité pour intervenir au plus vite. Ce secouriste n'est pas nécessairement un médecin. Il peut s'agir d'une personne ayant simplement suivi une formation de base aux premiers secours.

Ce secouriste ne sera pas non plus forcément une personne connaissant la zone géographique où est arrivé l'accident cardiaque. En particulier, ce secouriste n'aura pas systématiquement accès à la carte géographique des défibrillateurs cardiaques disponibles à proximité de l'endroit où est survenu l'accident cardiaque. Ce secouriste peut donc perdre de précieuses minutes dans cette recherche du défibrillateur cardiaque le plus proche ou d'un autre défibrillateur cardiaque relativement proche aussi.

Or, le délai d'intervention est critique pour la victime. En effet, si la défibrillation est effectuée rapidement, c'est-à-dire préférentiellement dans les toutes premières minutes après le début de la fibrillation, les chances de survie sont significativement augmentées.

Certes, souvent une cartographie des défibrillateurs cardiaques est accessible sur internet (par exemple sur un site publiant des données librement accessibles dites en « OpenData »). Le secouriste peut alors la consulter pour trouver le matériel d'intervention le plus proche de l'accident, mais il va perdre du temps dans cette recherche explicite, temps perdu qui risque d'être fatal à la victime.

### RESUME DE L'INVENTION

Le but de la présente invention est de fournir un procédé de gestion d'un parc de dispositifs de secours palliant au moins partiellement les inconvénients précités.

Plus particulièrement, l'invention vise à fournir un procédé de gestion d'un parc de dispositifs de secours qui va tendre à réduire le délai d'intervention des secouristes sur les victimes d'un problème de santé nécessitant l'utilisation d'un dispositif de secours non possédé par les secouristes.

Plus particulièrement, l'invention vise à fournir un procédé de gestion d'un parc de dispositifs de secours qui va mieux distribuer et mieux faire circuler l'information entre les différents éléments du réseau, à savoir serveur, appareil de surveillance et dispositifs de secours, et éventuellement également appareils de télécommunication, pour permettre de diminuer le temps de déplacement total avant intervention médicale, c'est-à-dire le temps nécessaire pour regrouper au niveau de l'appareil de surveillance de la victime, au moins un dispositif de secours et au moins un secouriste.

Plus particulièrement, l'invention vise à fournir un procédé de gestion d'un parc de dispositifs de secours qui va d'abord se concentrer sur le dispositif de secours et sur sa proximité avec l'appareil de surveillance à l'origine du signal ayant permis la localisation d'une victime, plutôt que sur les secouristes répertoriés et leur proximité avec l'appareil de surveillance à l'origine du signal ayant permis la localisation d'une victime, afin d'une part de réduire le temps de déplacement avant intervention et d'autre part de faciliter également l'intervention de secouristes même non répertoriés.

Le fait de se concentrer d'abord sur le dispositif de secours proche de l'appareil de surveillance à l'origine du signal ayant permis la localisation d'une victime, plutôt que sur le ou les secouristes disponibles au voisinage, ainsi que sur l'optimisation du trajet entre dispositif de secours et appareil de surveillance à l'origine du signal ayant permis la localisation d'une victime, en réalité plus critique que le trajet entre secouriste et appareil de surveillance à l'origine du signal ayant permis la localisation d'une victime, permet d'une part de réduire le temps de déplacement avant intervention proprement dite, et d'autre part de toucher en priorité un ou plusieurs secouristes proches du dispositif de secours plutôt que de l'appareil de surveillance à l'origine du signal ayant permis la localisation d'une victime.

A cette fin, la présente invention propose un procédé de gestion d'un parc de dispositifs de secours, comprenant : une étape de localisation d'un appareil de surveillance porté par une victime d'un problème de santé, une étape de localisation, dans le parc de dispositifs de secours, d'au moins un dispositif de secours proche de l'appareil de surveillance localisé, une étape d'alarme signalant au moins la position du dispositif de secours localisé, une étape de guidage indiquant, au niveau du dispositif de secours localisé, au moins la position de l'appareil de surveillance localisé.

A cette fin, la présente invention propose aussi un serveur de gestion d'un parc de dispositifs de secours, adapté pour réaliser : une étape de localisation d'un appareil de surveillance porté par une victime d'un problème de santé, une étape de localisation, dans le parc de dispositifs de secours, d'un dispositif de secours proche de l'appareil de surveillance localisé, une étape d'envoi, au dispositif de secours localisé, d'un ordre de déclenchement d'alarme signalant au moins la position du dispositif de secours localisé, une étape d'envoi, au dispositif de secours localisé, d'une instruction de guidage indiquant au moins la position de l'appareil de surveillance localisé.

A cette fin, la présente invention propose encore un dispositif de secours destiné à être disposé au sein d'un parc de dispositifs de secours, et adapté pour réaliser, lorsque ce dispositif de secours est localisé, par un serveur de gestion du parc de dispositifs de secours, comme proche d'un appareil de surveillance porté par une victime d'un problème de santé : une étape d'alarme, déclenchée sur ordre du serveur de gestion, signalant au moins la position de ce dispositif de secours localisé, une étape de guidage indiquant au moins la position de l'appareil de surveillance, après avoir reçu cette position de la part du serveur de gestion.

Suivant des modes de réalisation préférés, l'invention comprend une ou plusieurs des caractéristiques suivantes qui peuvent être utilisées séparément ou en combinaison partielle entre elles ou en combinaison totale entre elles, en association avec l'un quelconque des objets de l'invention précédemment décrits.

De préférence, le procédé de gestion d'un parc de dispositifs de secours comprend aussi une étape de localisation d'au moins un appareil de télécommunication porté par un secouriste proche du dispositif de secours localisé, et une étape d'envoi, à l'appareil de télécommunication localisé, au moins de la position du dispositif de secours localisé. Cela permet de toucher en priorité un secouriste proche du dispositif de secours, lequel sera donc à même de prendre ce dispositif de secours plus vite et de se diriger plus sûrement vers l'appareil de surveillance à l'origine du signal ayant permis la localisation d'une victime, plutôt qu'un secouriste proche de la victime mais qui ignore où et comment aller chercher un dispositif de secours.

De préférence, dans l'étape d'envoi, un parcours vers le dispositif de secours localisé est envoyé à l'appareil de télécommunication localisé, ce parcours étant de préférence recalculé en temps réel en fonction d'un éventuel déplacement de ce dispositif de secours localisé. Ainsi, le secouriste alerté, peut rallier au plus vite le dispositif de secours le plus proche de la victime, tout en raccourcissant son temps de parcours si le dispositif de secours est déjà en déplacement vers la victime.

De préférence, dans l'étape d'envoi, au moins la position de l'appareil de surveillance localisé est envoyée à l'appareil de télécommunication localisé, de préférence un parcours vers l'appareil de surveillance localisé est envoyé à l'appareil de télécommunication localisé. Ainsi, le secouriste peut disposer de toutes les informations de localisation sur son appareil de télécommunication, aussi bien celles concernant le dispositif de secours que celles concernant l'appareil de surveillance à l'origine du signal ayant permis la localisation d'une victime.

De préférence, dans l'étape d'alarme, une alarme visuelle et/ou une alarme sonore sont déclenchées au niveau du dispositif de secours localisé, et dans l'étape de guidage, au niveau du dispositif de secours localisé, au moins une position de l'appareil de surveillance localisé est indiquée, de préférence un parcours vers l'appareil de surveillance localisé est indiqué. Ainsi, d'une part tous les secouristes potentiels, situés au voisinage du dispositif de secours et donc le plus à même de l'amener le plus rapidement possible vers la victime, même s'ils ne sont pas répertoriés et même s'ils ne portent pas d'appareil de télécommunication, vont être touchés immédiatement, et d'autre part une fois arrivé ou arrivés au niveau du dispositif de secours, celui-ci prend le relais pour le ou les guider vers la victime.

De préférence, ledit parcours ou lesdits parcours sont le ou les plus rapides en temps de déplacement pour le dispositif de secours localisé et/ou pour l'appareil de télécommunication localisé, ce temps de parcours étant de préférence inférieur à une durée prédéterminée, cette durée prédéterminée étant encore plus de préférence inférieure à 10 minutes voire inférieure à 5 minutes. Il s'agit avantageusement d'optimiser le temps total de déplacement permettant à au moins un dispositif de secours et à au moins un secouriste de rallier le lieu de l'accident où se trouve la victime, que le dispositif de secours ait été amené au niveau de la victime par le secouriste qui va pratiquer l'intervention ou par quelqu'un d'autre. A l'intérieur des bâtiments ou en milieu urbain, ce temps de déplacement sera un temps de déplacement en marche, tandis qu'en milieu rural, ce pourra être un temps de déplacement en véhicule.

De préférence, dans l'étape de guidage et/ou dans l'étape d'envoi, une ou plusieurs autres informations complémentaires peuvent être indiquées, parmi lesquelles : la ou les positions du ou des autres dispositifs de secours localisés, et/ou la position du ou des autres appareils de télécommunication localisés, et/ou le profil de compétence médicale du ou des autres appareils de télécommunication localisés, et/ou l'identification de l'appareil de surveillance vers lequel se dirige le ou chaque dispositif de secours localisé, et/ou une confirmation de prise en charge par un ou plusieurs appareils de télécommunication localisés. Toutes ces informations complémentaires aident à optimiser la gestion de l'accident, permettant même par exemple d'utiliser à bon escient plusieurs secouristes de différents profils, par exemple à la fois un simple secouriste premiers soins arrivé plus tôt sur le lieu de l'accident et un médecin bien sûr plus qualifié mais qui n'arrivera que plus tard sur le lieu de l'accident, ou de réduire encore le temps de déplacement avant intervention dans le cas où une personne non secouriste se trouve située plus près qu'un secouriste du lieu de l'accident.

De préférence, dans l'étape de localisation de l'appareil de télécommunication, le ou les appareils de télécommunication localisés est ou sont le ou les appareils de télécommunication le ou les plus proches du dispositif de secours localisé, le ou les appareils de télécommunication localisés étant déterminés de préférence parmi un groupe prédéterminé d'appareils de télécommunication portés par des secouristes et répertoriés. Il s'agit de réduire encore le temps de déplacement avant intervention, et éventuellement d'avertir en cas de besoin au plus tôt des secouristes répertoriés même si ceux-ci ne sont pas dans le voisinage immédiat du dispositif de secours.

De préférence, les étapes du procédé se déroulent en parallèle avec au moins deux dispositifs de secours localisés et/ou avec au moins deux appareils de télécommunication localisés par dispositif de secours localisé. Cela augmente les chances d'avoir à temps près de la victime au moins un dispositif de secours et au moins un secouriste, malgré les imprévus sur leurs trajets.

De préférence, le déplacement du dispositif de secours localisé vers l'appareil de surveillance localisé et/ou l'arrivée du dispositif de secours localisé au niveau de l'appareil de surveillance localisé, sont indiqués à l'appareil de télécommunication localisé et/ou à d'autres appareils de télécommunication portés par des secouristes et alertés. Ainsi, le secouriste, grâce à son appareil de télécommunication, peut en temps réel, modifier son trajet en fonction de la position relative de la victime qui ne bouge plus et du dispositif de secours qui bouge vers la victime, et ceci, afin de réduire encore son temps de trajet pour rallier le lieu de l'accident en même temps ou presque en même temps que le dispositif de secours alors transporté par une personne non secouriste.

De préférence, l'étape de localisation d'un appareil de surveillance est précédée d'une étape d'identification de cet appareil de surveillance par analyse des données transmises par cet appareil de surveillance révélant un problème de santé chez la victime portant ledit appareil de surveillance. De préférence, l'appareil de surveillance envoie des données à un serveur qui gère le parc de dispositifs de secours dans lequel évolue cet appareil de surveillance, ledit serveur pouvant, au travers de l'analyse de ces données, identifier et localiser cet appareil de surveillance. Ainsi, le serveur gestionnaire d'une part peut lancer le procédé d'alerte au plus tôt après l'accident mais d'autre part seulement à bon escient. A bon escient, c'est-à-dire seulement s'il y a un véritable accident ou un vrai risque d'accident, et non pas à tout bout de champ ce qui nuirait, à la longue, à l'efficacité du procédé en lassant les différents intervenants devenus alors moins réactifs, car ayant été trop souvent sollicités sans raison valable. A titre d'alternative optionnelle, l'étape de localisation d'un appareil de surveillance est réalisée directement après transmission par l'appareil de surveillance de sa géolocalisation, ledit appareil de surveillance ayant réalisé lui-même une analyse de données révélant un problème de santé chez la victime portant ledit appareil de surveillance.

De préférence, deux appareils de télécommunication localisés sont mis en télécommunication l'un avec l'autre, le premier appareil de télécommunication localisé arrivé au niveau de l'appareil de surveillance localisé correspondant à un profil de compétence médicale inférieur à celui du deuxième appareil de télécommunication localisé non encore arrivé au niveau de l'appareil de surveillance localisé. Ainsi, cela permet par exemple d'utiliser à bon escient plusieurs secouristes de différents profils, notamment à la fois un simple secouriste premiers soins arrivé plus tôt sur le lieu de l'accident et un médecin bien sûr plus qualifié mais qui n'arrivera que plus tard sur le lieu de l'accident. Dans ce cas-là, en effet, le médecin, en route vers le lieu de l'accident, peut déjà instruire oralement le secouriste premiers soins qui se trouve déjà sur place sur les premiers gestes à faire en toute urgence, quitte à prendre ensuite le relais dès que lui aussi sera arrivé sur le lieu de l'accident.

De préférence, le dispositif de secours est un défibrillateur cardiaque. En effet, aujourd'hui, les parcs de défibrillateurs cardiaques sont développés et continuent à se développer, les accidents cardiaques de fibrillation sont graves mais peuvent être soignés par de simples secouristes premiers soins pourvu que ceux-ci arrivent à temps sur le lieu de l'accident.

Le dispositif de secours peut avantageusement également être tout autre dispositif de secours qu'un défibrillateur cardiaque qui serait distribué au sein d'un ou de plusieurs parcs de dispositifs de secours, qui pourrait soigner un accident grave mais à l'aide d'un simple secouriste premiers soins ou même d'un simple secouriste improvisé, c'est-à-dire que ce dispositif de secours serait d'utilisation très simple même pour du personnel non médical, mais qui nécessiterait toutefois une intervention rapide sur le lieu de l'accident.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES DESSINS

La figure 1 représente schématiquement un exemple de système adapté pour dérouler le procédé de gestion d'un parc de défibrillateurs cardiaques selon un mode de réalisation de l'invention.
La figure 2 représente schématiquement un exemple de déroulement du procédé de gestion d'un parc de défibrillateurs cardiaques selon un mode de réalisation de l'invention.

### DESCRIPTION DETAILLEE DE L'INVENTION

Dans toute la suite du texte, le dispositif de secours considéré sera un défibrillateur cardiaque, mais l'invention pourrait également s'appliquer à un autre type de dispositif de secours. Sans réduire la généralité d'application du procédé selon l'invention, l'appareil de télécommunication considéré sera un téléphone portable, de type usuel ou de type smartphone, une montre communicante, une tablette, un bip type bip de vigile, ou tout autre appareil de télécommunication adapté.

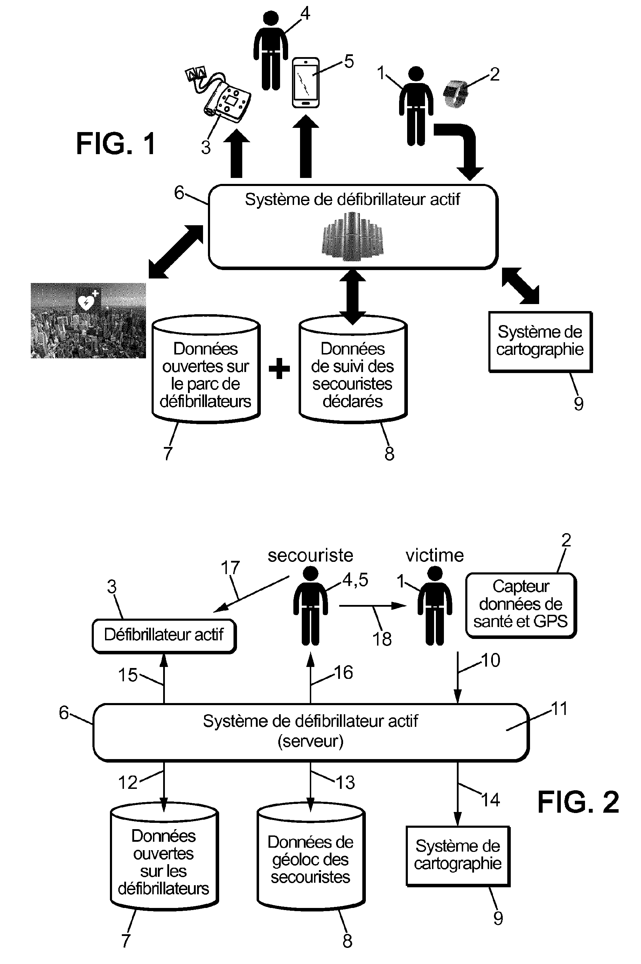

La figure 1 représente schématiquement un exemple de système adapté pour dérouler le procédé de gestion d'un parc de défibrillateurs cardiaques selon un mode de réalisation de l'invention.

Une victime potentielle 1 porte un appareil de surveillance 2. Un ou plusieurs secouristes potentiels 4 répertoriés portent chacun un téléphone portable 5. D'autres secouristes 4 improvisés peuvent participer, mais ils devront avoir été alertés par l'alarme au niveau du défibrillateur 3. Si un secouriste 4 ne porte pas son téléphone portable 5 ou ne l'a pas allumé, il ne pourra participer que comme secouriste improvisé alerté par l'alarme au niveau du défibrillateur 3. La victime 1 comme le ou les secouristes 4 évoluent dans des endroits voisins au sein d'un parc de défibrillateurs 3. Le parc de défibrillateurs 3 est géré par un serveur 6 lequel peut accéder à une base de données 7 contenant la distribution des défibrillateurs 3 au sein du parc, à une base de données 8 répertoriant plusieurs secouristes 4 déclarés, à un système de cartographie 9 permettant d'aider à localiser en temps réel aussi bien l'appareil de surveillance 2 de la victime 1 que les téléphones portables 5 des secouristes 4 et que le ou les défibrillateurs 3 en déplacement vers le lieu de l'accident où se trouve la victime 1.

L'appareil de surveillance 2 de la victime potentielle 1 d'un accident cardiaque comprend un capteur de données de santé et un capteur de géolocalisation de type GPS (GPS pour « Global Positioning System » en langue anglaise) ainsi qu'un module de télécommunication.

Le capteur de données de santé, parmi lesquelles notamment la fréquence cardiaque et la tension artérielle, est adapté pour transmettre ces données de santé périodiquement vers le serveur 6. Un tel capteur est par exemple une montre « Apple Iwatch » (marque déposée). L'envoi de ces données de santé au serveur 6 peut alors s'effectuer à l'aide d'une application mobile.

Le capteur de géolocalisation permet de relever les coordonnées de la victime 1 et de les envoyer vers le serveur 6 par l'intermédiaire du module de télécommunication par exemple à l'aide d'une application mobile.

Le téléphone portable 5 du secouriste 4 intègre une application mobile permettant de suivre la localisation de ce secouriste 4 et de lui notifier la survenue d'un accident à proximité.

Le défibrillateur actif 3 comprend une alarme et un afficheur de guidage.

L'alarme comprend avantageusement à la fois une sonnerie et une lampe clignotante, tous deux pilotables à distance depuis le serveur 6.

L'afficheur de guidage comprend un système d'orientation vers la l'appareil de surveillance 2 de la victime 1, qui est pilotable à distance par le serveur 6. Le serveur 6 envoie au défibrillateur actif 3 les étapes du parcours à suivre depuis ce défibrillateur actif 3 pour arriver sur le lieu de l'accident, au niveau de l'appareil de surveillance 2 de la victime 1. Le défibrillateur actif 3 est le défibrillateur 3 qui a été localisé par le serveur 6. Ce système d'orientation comprend lui-même un système de géolocalisation de type GPS pour adapter le parcours lors du cheminement du secouriste 4 vers le lieu de l'accident, le calcul d'adaptation du parcours en permanence étant effectué par le serveur 6.

Le serveur 6 est avantageusement un serveur informatique virtuel ou réel hébergeant la logique métier du système de gestion du parc de défibrillateurs actifs 3. Dans cette logique métier, sont intégrées plusieurs fonctions. Une première fonction est la détection d'anomalie cardiaque grave à partir des données de santé reçue de l'appareil de surveillance 2 d'une victime potentielle 1, ou de chaque appareil de surveillance 2 appartenant respectivement aux différentes victimes potentielles 1. Une deuxième fonction est la recherche du défibrillateur actif 3 qui est le plus proche de l'appareil de surveillance 2 à l'origine du signal ayant permis la localisation de la victime 1. La troisième fonction est le déclenchement, à distance, de l'alarme du défibrillateur actif 3 localisé comme étant le plus proche du lieu de l'accident. La quatrième fonction est le calcul du parcours le plus rapide entre ce défibrillateur 3 localisé et l'appareil de surveillance 2 porté par la victime 1 qui se trouve sur le lieu de l'accident.

Le serveur 6 communique aussi avec une base de données 7 ouverte cartographiant le parc de défibrillateurs 3 installés (par exemple du type « OpenData », marque déposée).

Le serveur 6 communique aussi avec une base de données 8 de géolocalisation et de contact des secouristes 4 déclarés qui y sont donc répertoriés.

Le serveur 6 communique aussi avec un système de cartographie 9 accessible depuis une interface programmatique (par exemple de type « Google Maps », marque déposée).

La figure 2 représente schématiquement un exemple de déroulement du procédé de gestion d'un parc de défibrillateurs cardiaques selon un mode de réalisation de l'invention. Les différents « temps », définis ci-après, soit se succèdent, rapidement en général, soit certains d'entre eux peuvent être effectués simultanément avec les temps voisins, voir intervertis entre eux. Notamment, certaines des étapes qui peuvent être effectuées en parallèle, c'est-à-dire simultanément, peuvent aussi être inversées.

De la même manière que pour la figure 1, une victime potentielle 1 porte un appareil de surveillance 2. Un ou plusieurs secouristes potentiels 4 répertoriés portent chacun un téléphone portable 5. La victime 1 comme le ou les secouristes 4 évoluent dans des endroits voisins au sein d'un parc de défibrillateurs 3. Le parc de défibrillateurs 3 est géré par un serveur 6 lequel peut accéder à une base de données 7 contenant la distribution des défibrillateurs 3 au sein du parc, à une base de données 8 répertoriant plusieurs secouristes 4 déclarés, à un système de cartographie 9 permettant d'aider à localiser en temps réel aussi bien l'appareil de surveillance 2 de la victime 1 que les téléphones portables 5 des secouristes 4 et que le ou les défibrillateurs 3 en déplacement vers le lieu de l'accident où se trouve la victime 1.

Dans un premier temps 10, l'appareil de surveillance 2 collecte et envoie des données de santé et des données de géolocalisation concernant la victime 1. Ces données proviennent respectivement du capteur de données de santé et du capteur de géolocalisation que comprend l'appareil de surveillance 2.

Dans un deuxième temps 11, le serveur 6 procède à la détection et à l'identification de la victime 1 d'un accident cardiaque grave. Pour cela, le serveur 6 détermine que la victime 1 a subi un accident cardiaque grave ou est en risque immédiat de subir un accident cardiaque grave, à partir de l'analyse des données reçues du capteur de santé en étant envoyées par l'appareil de surveillance 2. Le serveur 6 procède aussi à la localisation de l'appareil de surveillance 2, par exemple à partir des coordonnées de géolocalisation fournies par l'appareil de surveillance 2.

Dans un troisième temps 12, le serveur 6 recherche dans la base de données 7 contenant la distribution des défibrillateurs au sein du parc, le défibrillateur 3 le plus proche de l'appareil de surveillance 2 qui se trouve sur le lieu de l'accident subi par la victime 1.

Dans un quatrième temps 13, le serveur 6 recherche dans la base de données 8 répertoriant plusieurs secouristes 4 déclarés, le ou les secouristes 4 les plus proches de la victime 1, en cherchant leurs téléphones portables 5 et en sélectionnant le ou les téléphones portables 5 de ces secouristes 4 qui se trouvent au voisinage du lieu de l'accident, c'est-à-dire qui sont proches de l'appareil de surveillance 2.

Dans un cinquième temps 14, le serveur 6 d'une part récupère le parcours le plus rapide entre le défibrillateur 3 localisé comme le plus proche et l'appareil de surveillance 2 de la victime 1, sur le système de cartographie 9, et d'autre part récupère le ou les parcours les plus rapides entre le ou les téléphones portables 5 des secouristes 4 et l'appareil de surveillance 2 de la victime 1, sur ce même système de cartographie 9.

Dans un sixième temps 15, le serveur 6 déclenche à distance l'alarme, par exemple une sonnerie et une lumière clignotante, sur le défibrillateur localisé 3 pour alerter les secouristes 4 se trouvant aux alentours, qu'ils soient déclarés et répertoriés sur la base de données 7 et qu'ils puissent ensuite être notifiés par l'intermédiaire de leurs téléphones portables 5 ou bien qu'ils soient non déclarés et qu'ils passent simplement au voisinage du défibrillateur localisé 3. Simultanément, le serveur 6 envoie au défibrillateur localisé 3 des étapes du parcours pour rejoindre la victime 1 sur le lieu de l'accident, c'est-à-dire avantageusement un parcours depuis ce défibrillateur localisé 3 vers l'appareil de surveillance 2 de cette victime 1.

Dans un septième temps 16, le serveur 6 notifie les secouristes 4 déclarés se trouvant aux alentours, c'est-à-dire ceux qui sont répertoriés sur la base de données 7 et qui sont joignables par l'intermédiaire de leurs téléphones portables 5, par exemple via une application mobile sur smartphone si leurs téléphones portables 5 la portent. A ces téléphones portables 5 aussi sont envoyées des étapes du parcours pour rejoindre le défibrillateur localisé 3 et la victime 1 sur le lieu de l'accident, c'est-à-dire avantageusement un premier parcours depuis leur position jusqu'au défibrillateur localisé 3 et un deuxième parcours depuis ce défibrillateur localisé 3 vers l'appareil de surveillance 2 de cette victime 1. Ces premier et deuxième parcours peuvent avantageusement être recalculés et mis à jour en temps réel par le serveur 6, notamment en cas de déplacement du défibrillateur localisé 3 emporté par un secouriste 4, déclaré ou non.

Dans un huitième temps 17, le ou les secouristes 4 notifiés par l'intermédiaire de leurs téléphones portables 5 et/ou bien un ou plusieurs secouristes 4 non déclarés ayant entendu l'alarme déclenchée au niveau du défibrillateur localisé 3, va ou vont récupérer le défibrillateur localisé 3, en étant guidés par la sonnerie et/ou la lumière clignotante émises par le défibrillateur localisé 3 ou encore par leur application mobile pour les secouristes 4 ayant été notifiés par le serveur 6.

Dans un neuvième temps 18, le secouriste 4 arrivé le premier au niveau du défibrillateur localisé 3 et l'ayant emporté, est guidé vers l'appareil de surveillance 2 de la victime 1, c'est-à-dire vers le lieu de l'accident, par l'intermédiaire du parcours affiché sur un afficheur du défibrillateur localisé 3 et/ou par l'intermédiaire d'une interface vocale du défibrillateur localisé 3 et/ou par l'intermédiaire d'une application mobile sur son téléphone portable 5 lorsque ce secouriste a été notifié par le serveur 6.

Le défibrillateur localisé 3 peut également comporter une fonction de gestion du retour de ce défibrillateur localisé 3, laquelle fonction peut calculer par exemple l'itinéraire vers la station de retour la plus proche.

Bien entendu, la présente invention n'est pas limitée aux exemples et au mode de réalisation décrits et représentés, mais elle est susceptible de nombreuses variantes accessibles à l'homme de l'art.

## Revendications

1. Procédé de gestion d'un parc de dispositifs de secours (3), comprenant :
- une étape de localisation (11) d'un appareil de surveillance (2) porté par une victime (1) d'un problème de santé,
- une étape de localisation (12), dans le parc de dispositifs de secours (3), d'au moins un dispositif de secours (3) proche de l'appareil de surveillance (2) localisé,
- une étape d'alarme (15) signalant au moins la position du dispositif de secours (3) localisé,
- une étape de guidage (18) indiquant, au niveau du dispositif de secours (3) localisé, au moins la position de l'appareil de surveillance (2) localisé.

2. Procédé de gestion d'un parc de dispositifs de secours (3) selon la revendication 1, **caractérisé en ce qu'**il comprend aussi une étape de localisation (13) d'au moins un appareil de télécommunication (5) porté par un secouriste (4) proche du dispositif de secours (3) localisé, et une étape d'envoi (16), à l'appareil de télécommunication (5) localisé, au moins de la position du dispositif de secours (3) localisé.

3. Procédé de gestion d'un parc de dispositifs de secours (3) selon la revendication 2, **caractérisé en ce que**, dans l'étape d'envoi (16), un parcours vers le dispositif de secours (3) localisé est envoyé à l'appareil de télécommunication (5) localisé, ce parcours étant de préférence recalculé en temps réel en fonction d'un éventuel déplacement de ce dispositif de secours (3) localisé.

4. Procédé de gestion d'un parc de dispositifs de secours (3) selon la revendication 2 ou 3, **caractérisé en ce que**, dans l'étape d'envoi (16), au moins la position de l'appareil de surveillance (2) localisé est envoyée à l'appareil de télécommunication (5) localisé, de préférence un parcours vers l'appareil de surveillance (2) localisé est envoyé à l'appareil de télécommunication (5) localisé.

5. Procédé de gestion d'un parc de dispositifs de secours selon l'une quelconque des revendications précédentes, **caractérisé en ce que**, dans l'étape d'alarme (15), une alarme visuelle et/ou une alarme sonore sont déclenchées au niveau du dispositif de secours (3) localisé, et dans l'étape de guidage (18), au niveau du dispositif de secours (3) localisé, au moins une position de l'appareil de surveillance (2) localisé est indiquée, de préférence un parcours vers l'appareil de surveillance (2) localisé est indiqué.

6. Procédé de gestion d'un parc de dispositifs de secours (3) selon l'une quelconque des revendications précédentes, **caractérisé en ce que** ledit parcours ou lesdits parcours sont le ou les plus rapides en temps de déplacement pour le dispositif de secours (3) localisé et/ou pour l'appareil de télécommunication (5) localisé, ce temps de parcours étant de préférence inférieur à une durée prédéterminée, cette durée prédéterminée étant encore plus de préférence inférieure à 10 minutes voire inférieure à 5 minutes.

7. Procédé de gestion d'un parc de dispositifs de secours (3) selon l'une quelconque des revendications précédentes, **caractérisé en ce que**, dans l'étape de guidage (18) et/ou dans l'étape d'envoi (16), une ou plusieurs autres informations complémentaires peuvent être indiquées, parmi lesquelles : la ou les positions du ou des autres dispositifs de secours (3) localisés, et/ou la position du ou des autres appareils de télécommunication (5) localisés, et/ou le profil de compétence médicale du ou des autres appareils de télécommunication (5) localisés, et/ou l'identification de l'appareil de surveillance (2) vers lequel se dirige le ou chaque dispositif de secours (3) localisé, et/ou une confirmation de prise en charge par un ou plusieurs appareils de télécommunication (5) localisés.

8. Procédé de gestion d'un parc de dispositifs de secours (3) selon l'une quelconque des revendications précédentes, **caractérisé en ce que**, dans l'étape de localisation (13) de l'appareil de télécommunication (5), le ou les appareils de télécommunication (5) localisés est ou sont le ou les appareils de télécommunication (5) le ou les plus proches du dispositif de secours (3) localisé, le ou les appareils de télécommunication (5) localisés étant déterminés de préférence parmi un groupe prédéterminé d'appareils de télécommunication (5) portés par des secouristes (4) et répertoriés.

9. Procédé de gestion d'un parc de dispositifs de secours (3) selon l'une quelconque des revendications précédentes, **caractérisé en ce que** les étapes du procédé se déroulent en parallèle
avec au moins deux dispositifs de secours (3) localisés,
et/ou avec au moins deux appareils de télécommunication (5) localisés par dispositif de secours (3) localisé, et de préférence **en ce que** deux appareils de télécommunication (5) localisés sont mis en télécommunication l'un avec l'autre, le premier appareil de télécommunication (5) localisé arrivé au niveau de l'appareil de surveillance (2) localisé correspondant à un profil de compétence médicale inférieur à celui du deuxième appareil de télécommunication (5) localisé non encore arrivé au niveau de l'appareil de surveillance (2) localisé.

10. Procédé de gestion d'un parc de dispositifs de secours (3) selon l'une quelconque des revendications précédentes, **caractérisé en ce que** le déplacement du dispositif de secours (3) localisé vers l'appareil de surveillance (2) localisé et/ou l'arrivée du dispositif de secours (3) localisé au niveau de l'appareil de surveillance (2) localisé, sont indiqués à l'appareil de télécommunication (5) localisé et/ou à d'autres appareils de télécommunication (5) portés par des secouristes (4) et alertés.

11. Procédé de gestion d'un parc de dispositifs de secours (3) selon l'une quelconque des revendications précédentes,
**caractérisé en ce que** l'étape de localisation (11) d'un appareil de surveillance (2) est précédée d'une étape d'identification (11) de cet appareil de surveillance (2) par analyse des données transmises par cet appareil de surveillance (2) révélant un problème de santé chez la victime (1) portant ledit appareil de surveillance (2),
ou **en ce que** l'étape de localisation (11) d'un appareil de surveillance (2) est réalisée directement après transmission par l'appareil de surveillance (2) de sa géolocalisation, ledit appareil de surveillance (2) ayant réalisé lui-même une analyse de données révélant un problème de santé chez la victime (1) portant ledit appareil de surveillance (2).

12. Procédé de gestion d'un parc de dispositifs de secours (3) selon l'une quelconque des revendications précédentes, **caractérisé en ce que** l'appareil de surveillance (2) envoie des données à un serveur (6) qui gère le parc de dispositifs de secours (3) dans lequel évolue cet appareil de surveillance (2), ledit serveur (6) pouvant, au travers de l'analyse de ces données, identifier et localiser cet appareil de surveillance (2).

13. Procédé de gestion d'un parc de dispositifs de secours (3) selon l'une quelconque des revendications précédentes, **caractérisé en ce que** le dispositif de secours (3) est un défibrillateur cardiaque.

14. Serveur de gestion d'un parc de dispositifs de secours (3), adapté pour réaliser :
- une étape de localisation (11) d'un appareil de surveillance (2) porté par une victime (1) d'un problème de santé,
- une étape de localisation (12), dans le parc de dispositifs de secours (3), d'un dispositif de secours (3) proche de l'appareil de surveillance (2) localisé,
- une étape d'envoi (15), au dispositif de secours (3) localisé, d'un ordre de déclenchement d'alarme signalant au moins la position du dispositif de secours (3) localisé,
- une étape d'envoi (15), au dispositif de secours localisé (3), d'une instruction de guidage indiquant au moins la position de l'appareil de surveillance (2) localisé.

15. Dispositif de secours destiné à être disposé au sein d'un parc de dispositifs de secours (3), et adapté pour réaliser, lorsque ce dispositif de secours (3) est localisé, par un serveur (6) de gestion du parc de dispositifs de secours (3), comme proche d'un appareil de surveillance (2) porté par une victime (1) d'un problème de santé :
- une étape d'alarme (15), déclenchée sur ordre du serveur (6) de gestion, signalant au moins la position de ce dispositif de secours (3) localisé,
- une étape de guidage (18) indiquant au moins la position de l'appareil de surveillance (2), après avoir reçu cette position de la part du serveur (6) de gestion.
